# EUROPEAN PATENT APPLICATION

(11) **EP 4 670 637 A1**
(43) Date of publication of application: **31.12.2025**
(21) Application number: 24184869.6
(22) Date of filing: 27.06.2024
(51) Int. Cl.: A61B 6/04, A61N 5/10

(54) **MULTI-SENSOR SYSTEM FOR PATIENT SETUP GUIDANCE**

(71) Applicant: Koninklijke Philips N.V., 5656 AG Eindhoven (NL)
(72) Inventor: KRUEGER, Sascha, Eindhoven (NL)
(74) Representative: Philips Intellectual Property & Standards

(57) **Abstract**

The present invention relates to a sensor arrangement for patient setup guidance to be embedded in a medical imaging or treatment system, the sensor arrangement comprising a plurality of sensors, wherein at least a first sensor of the plurality of sensors comprises a field of view of the first sensor having an aspect ratio unequal to 1, thus comprising a first axis of the field of view of the first sensor and a second axis of the field of view of the first sensor, wherein the field of view of the first sensor is larger in the direction of the first axis than in the direction of the second axis, and wherein the plurality of sensors are arranged along a line parallel to the direction of the second axis of the field of view of the first sensor.

## Description

### FIELD OF THE INVENTION

The present invention relates to a sensor arrangement for patient setup guidance to be embedded in a medical imaging or treatment system, and a medical imaging or treatment system comprising the sensor arrangement.

### BACKGROUND OF THE INVENTION

In clinical workflows, preparing a patient and setting up the patient for a medical imaging examination is a complex task requiring trained personnel. However, hospital department schedules are increasingly tight, and medical-technical assistants MTA doing this task have to navigate pressure of time while trying to preserve a positive patient experience, safety, and diagnostic image quality. The usage of cameras has been proposed to address this issue by providing guidance and automation where appropriate. Various medical imaging systems use sensors to assist hospital staff in preparing a patient for image taking and to reduce the throughput time per patient examination for workflow optimization. Arrangements with multiple sensors are beneficial, providing multiple complementary views with different sensor or data properties. In particular, depth image sensors have shown to be advantageous by providing accurate depth ranging. However, sensor choice and arrangement is not trivial or obvious and depends on the intended application. In common arrangements, such sensors typically can be ceiling mounted and look down onto the patient support table and the patient. A set of multiple sensors might be applied, including sensors of different types.

Current commercially available combined sensor arrangements are typically not optimized for a specific application, e.g., patient setup workflow support, and even less for dedicated features thereof. Instead, sensor arrangement on such systems is usually a reasonable compromise for a generic range of applications and follows practical requirements, like cost, device form factor, or thermal issues.

The inventors of the present invention have thus found that it would be advantageous to have an improved sensor arrangement that is optimized for the specific application of patient setup for imaging or treatment.

### SUMMARY OF THE INVENTION

It is an object of the present invention to provide a more dedicated and favorable sensor arrangement that allows minimization of individual sensor data deficits, and maximization of the integral sensing capability of the arrangement for a given application.

The object of the present invention is solved by the subject matter of the independent claims, wherein further embodiments are incorporated in the dependent claims.

The described embodiments similarly pertain to the sensor arrangement for patient setup guidance to be embedded in a medical imaging or treatment system, and the medical imaging or treatment system. The embodiments described further may be combined in any possible way. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method, unless explicitly mentioned to the contrary hereinafter.

According to a first aspect of the invention, there is provided a sensor arrangement for patient setup guidance to be embedded in a medical imaging or treatment system, the sensor arrangement comprising a plurality of sensors, wherein at least a first sensor of the plurality of sensors comprises a field of view of the first sensor having an aspect ratio unequal to 1, thus comprising a first axis of the field of view of the first sensor and a second axis of the field of view of the first sensor, wherein the field of view of the first sensor is larger in the direction of the first axis than in the direction of the second axis, and wherein the plurality of sensors are arranged along a line parallel to the direction of the second axis of the field of view of the first sensor.

Thus, a preferred sensor arrangement for sensors having an aspect ratio unequal 1, i.e. having both a short axis and a long axis, is proposed. Such an arrangement advantageously optimizes the field of view of the sensors. At the same time data deficits are minimized, as the sensor arrangement comprises multiple sensors, which are stacked, i.e., placed alongside each other, along their short axis. This arrangement avoids or at least reduces visibility differences between the different sensors along the patient head-feet axis along which the extent of the patient is largest. In such an embodiment, a mapping, synchronization, un-distortion and gridding reconstruction to map all sensors to a virtual main camera, which may be located at one main sensor, e.g. an RGB sensor, is preferred.

With the proposed multi-sensor arrangement mounted in a defined relative position and orientation with respect to a patient setup area, advantageously the acquisition of images during the patient preparation is improved, and guidance through the process of examination setup and evaluation after the examination can be easily automated. Reconstruction of the data acquired with the proposed sensor arrangement including spatio-temporal alignment is improved, and the computation of setup and examination parameters from the data is enhanced.

Thus, the gist of the invention is to have a workflow supporting sensor arrangement for a medical imaging or treatment device which can be mounted above the patient support table, and which comprises at least one sensor having a long axis and a short axis, wherein the long axis of the sensor is parallel to a longitudinal direction of the patient table, corresponding to the head-feet direction of a patient placed on the table for examination. Further sensors of the sensor arrangement are arranged in a line parallel to the short axis of the at least one sensor.
In an embodiment of the invention, the first axis of the field of view of the first sensor is perpendicular to the second axis of the field of view of the first sensor. The sensor may therefore have a rectangular field of view with matrix-shaped pixel alignment.

In an embodiment of the invention, a central axis of the field of view of the first sensor is oriented essentially perpendicular to the first axis and to the second axis. This enables the sensor to look onto the patient in a top-down direction, while the field of view covers the whole patient.

In an embodiment of the invention, the central axis corresponds to an imaging direction of the first sensor.

In an embodiment of the invention, each sensor of the plurality of sensors comprises a field of view having an aspect ratio unequal to 1, thus comprising a first axis of the field of view and a second axis of the field of view, wherein the field of view is larger in the direction of the first axis than in the direction of the second axis, and each sensor of the plurality of sensors is arranged along the line parallel to the direction of the second axis of the field of view of the first sensor such that the first axis of the field of view of the respective sensor is parallel to the direction of the first axis of the field of view of the first sensor.

In an embodiment of the invention, each one of the plurality of sensors is selected from the group consisting of an optical camera, an RGB camera, a depth sensing device, a time-of-flight sensor, a radar sensor, a lidar sensor, a thermal sensor, or an infrared camera. For example, if the sensor arrangement includes a depth sensor, a particularly advantageous approach can be provided to reconstruct steep edges, like, for example, table sides, and structures of a size in the order of the camera resolution, like cables.

In an embodiment of the invention, each sensor of the plurality of sensors is of a different type than any other sensor of the plurality of sensors. However, also two sensors of a same type may be advantageously provided in the proposed configuration and orientation of the sensor arrangement.

In an embodiment of the invention, the first sensor is an RGB sensor.

In an embodiment of the invention, the plurality of sensors are arranged along the line parallel to the direction of the second axis of the field of view of the first sensor such that a distance between each pair of adjacent sensors of the plurality of sensors is minimized.

In an embodiment of the invention, the distance between each pair of adjacent sensors of the plurality of sensors is smaller than 50 cm, preferably smaller than 30 cm, even more preferably smaller than 10 cm.

In an embodiment of the invention, the aspect ratio of the field of view of the first sensor is larger than 1.2:1, preferably larger than 1.5:1; even more preferably larger than 2:1.

In an embodiment of the invention, the sensor arrangement further comprises a control unit configured for receiving data from each of the plurality of sensors and mapping the received data to a virtual camera position, thereby providing a combined image.

According to another aspect of the invention, there is provided a medical imaging or treatment system comprising a patient bed configured for receiving a patient for imaging or treatment, and a sensor arrangement according to any one of the preceding embodiments, wherein the sensor arrangement is positioned above the patient bed and configured for imaging the patient, and wherein the first axis of the field of view of the first sensor is aligned parallel to a head-foot direction of the patient bed and the second axis of the field of view of the first sensor is aligned perpendicular to the head-foot direction of the patient bed.

In an embodiment of the invention, the sensor arrangement is frontally facing the patient bed and is configured for imaging the patient with each sensor of the plurality of sensors.

In an embodiment of the invention, a field of view of each sensor of the plurality of sensors encompasses the patient bed.

Thus, the benefits provided by any of the above aspects equally apply to all of the other aspects and vice versa.

In summary, the invention relates to a sensor arrangement for patient setup guidance to be embedded in a medical imaging or treatment system, the sensor arrangement comprising a plurality of sensors, wherein at least a first sensor of the plurality of sensors comprises a field of view of the first sensor having an aspect ratio unequal to 1, thus comprising a first axis of the field of view of the first sensor and a second axis of the field of view of the first sensor, wherein the field of view of the first sensor is larger in the direction of the first axis than in the direction of the second axis, and wherein the plurality of sensors are arranged along a line parallel to the direction of the second axis of the field of view of the first sensor.

One of the advantages of embodiments of the present invention is that a sensor arrangement is provided that is optimized for the specific application of patient setup workflow support. Further, individual sensor data deficits are minimized, and the integral sensing capability of the sensor arrangement can be maximized for a given application. The proposed sensor arrangement is particularly advantageous for camera-based workflow solutions for magnetic resonance tomography or computed tomography. However, in principle, it can be applied to all modalities, especially tomographic imaging modalities or therapy planning, but also to digital X-ray radiogrammetry or image-guided therapy.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1A shows a sensor arrangement for patient setup guidance to be embedded in a medical imaging or treatment system according to an embodiment of the invention.
Fig. 1B shows a first sensor of the sensor arrangement for patient setup guidance to be embedded in a medical imaging or treatment system according to an embodiment of the invention.
Fig. 2 shows a medical imaging or treatment system comprising the sensor arrangement for patient setup guidance according to an embodiment of the invention.
Fig. 3A shows an example of a depth image mapped to an RGB image acquired with a sensor arrangement according to an embodiment of the invention.
Figs 3B-D show a stable reconstruction of thin objects in a depth image acquired with a sensor arrangement according to an embodiment of the invention.

### DETAILED DESCRIPTION OF EMBODIMENTS

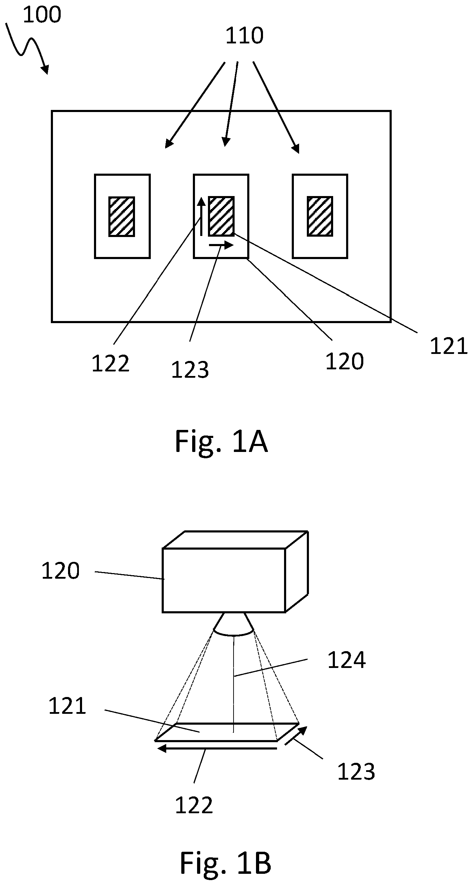

Fig. 1A shows a sensor arrangement 100 for patient setup guidance to be embedded in a medical imaging or treatment system 200 according to an embodiment of the invention. The sensor arrangement 100 comprises a plurality of sensors 110 arranged adjacent to each other in a line. Preferably each sensor, and in particular a first sensor 120, has a field of view with a rectangular or elongate shape, i.e., with an aspect ratio unequal to 1. Thus, the field of view 121 of the first sensor 120 comprises a first axis 122 and a second axis 123, and the field of view 121 of the first sensor 120 is larger in the direction of the first axis 122 than in the direction of the second axis 123. The line along which the plurality of sensors are arranged is parallel to the direction of the second axis 123 of the field of view 121 of the first sensor 120.

This arrangement advantageously optimizes the field of view of the sensors and helps to minimize data deficits, as the sensor arrangement 100 comprises multiple sensors, which are stacked, i.e., placed alongside each other, along their short axis. This arrangement avoids or at least reduces visibility differences between the different sensors along the patient head-feet axis along which the extent of the patient is largest. Thus, a mapping, synchronization, un-distortion and gridding reconstruction to map all sensors to a virtual main camera, which may be located at one main sensor, e.g. an RGB sensor, is advantageously enabled and guidance through the process of examination setup and evaluation after the examination can be easily automated.

The first axis 122 of the field of view of the first sensor may be perpendicular to the second axis 123 of the field of view of the first sensor. Both the first axis and the second axis may be oriented horizontally. The sensor may therefore have a rectangular field of view with matrix-shaped pixel alignment, and may look down onto a patient in a top-down imaging direction along a central, vertical axis.

Preferably, each sensor of the plurality of sensors has a field of view with an aspect ratio unequal to 1, and is arranged such that the longer axis of the field of view is oriented in a head-to-foot direction of the patient to be imaged. The sensors may be, for example, an optical camera, an RGB camera, a depth sensing device, a time-of-flight sensor, a radar sensor, a lidar sensor, a thermal sensor, or an infrared camera. Preferably, all sensors of the plurality of sensors 110 are of a different type, but there may be also sensors of an identical type in the sensor arrangement 100.

The plurality of sensors are arranged along the line parallel to the direction of the second axis of the field of view of the first sensor such that a distance between each pair of adj acent sensors of the plurality of sensors is minimized. Preferably, the distance between each pair of adjacent sensors of the plurality of sensors is smaller than 50 cm, preferably smaller than 30 cm, even more preferably smaller than 10 cm. The aspect ratio of the field of view of the first sensor 120 as well as of the other sensors may be larger than 1.2:1, preferably larger than 1.5:1; even more preferably larger than 2:1.

The sensor arrangement 100 may further comprise a control unit configured for receiving data from each of the plurality of sensors and mapping the received data to a virtual camera position, thereby providing a combined image.

Fig. 1B shows a first sensor 120 of the sensor arrangement 100 for patient setup guidance to be embedded in a medical imaging or treatment system 200 according to an embodiment of the invention. This Figure illustrates the relationship between the first sensor 120, the field of view 121 of the first sensor, and a central axis 124 of the imaging direction or field of view and the first axis 122 and the second axis 123 of the field of view. The first axis 122, the second axis 123 and the central axis 124 are preferably all perpendicular to each other.

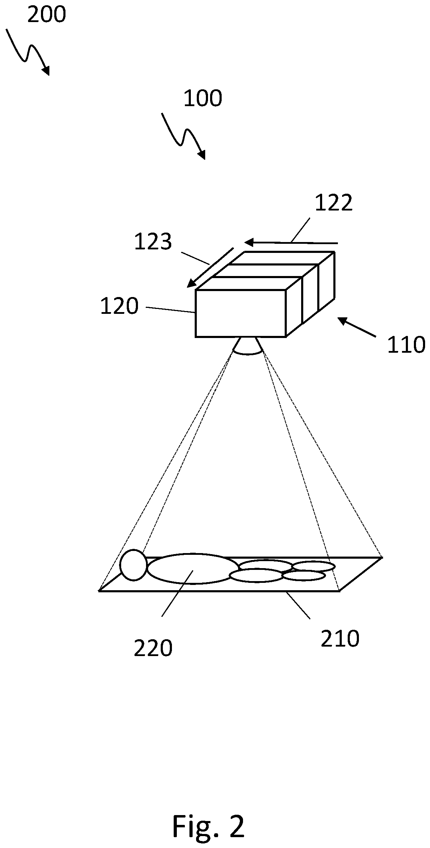

Fig. 2 shows a medical imaging or treatment system 200 comprising the sensor arrangement 100 for patient setup guidance according to an embodiment of the invention. The medical imaging or treatment system 200 comprises a patient bed 210 configured for receiving a patient 220 for imaging or treatment, and a sensor arrangement 100 for patient setup guidance as described above. The sensor arrangement 100 is positioned above the patient bed 210 and is configured for imaging the patient 220, and the first axis 122 of the field of view of the first sensor 120 is aligned parallel to a head-foot direction of the patient 220 or patient bed 210 and the second axis 123 of the field of view of the first sensor 120 is aligned perpendicular to the head-foot direction of the patient bed 220. The sensors of the plurality of sensors 110 are arranged in a line perpendicular to the head-foot direction, and thus parallel to the second axis of the field of view. The sensor arrangement 100 may be frontally facing the patient bed and may be configured for imaging the patient with each sensor of the plurality of sensors, such that a field of view of each sensor of the plurality of sensors encompasses the patient bed 210 and thus the patient 220.

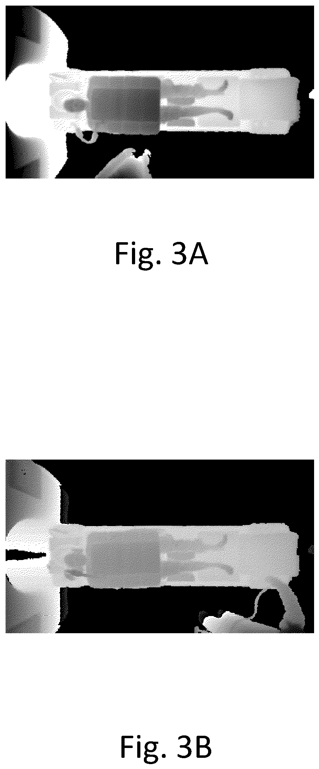

Fig. 3A shows an example of a depth image mapped to an RGB image acquired with a sensor arrangement 100 according to an embodiment of the invention, which is essentially free of any shadows along the head-foot axis due to avoidance of displacements of the image sensors along this axis. An arbitrary number of image sensors of which those with an aspect ratio different from 1 are oriented with their long axis along patient head-foot axis. All sensors are aligned preferably precisely with their short axis along the patient left-right axis. For patient workflow solutions, for example, it is advantageous to have the sensors aligned with their long-axis along the patient head-feet axis to make optimum use of the sensor field of view. At the same time, it is preferable to stack multiple such-oriented sensors along the short left-right axis of the patient to maximize the overlap of the visible parts in the relevant patient area. Reconstruction of the data acquired with the proposed sensor arrangement including spatio-temporal alignment is improved, and the computation of setup and examination parameters from the data is enhanced. Alignment of the sensors short axes avoids visibility differences along the patient head-foot axis along which the extent of the patient is largest. Stacking of sensors' short axis along short patient left-right axis may mean accepting some but rather minor shadowing in left-right axis due to the smaller spatial extent of the patient in this dimension. However, this is not detrimental to the use of a signal-conserving reconstruction, mapping and gridding algorithm.

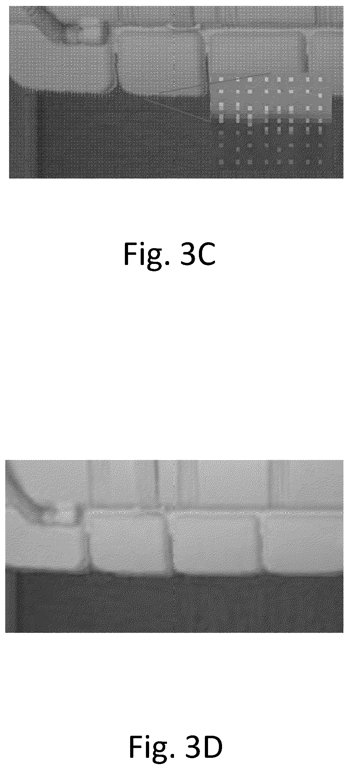

Fig. 3B-D show a stable reconstruction of thin objects in a depth image acquired with a sensor arrangement 100 according to an embodiment of the invention. Especially for thin objects, that may be in the order of the sensor resolution like cables, beneficial sensor alignment and respective reconstruction allows to recover the signal while it is not or only partially visible with current commercial systems. A mapping, synchronization, un-distortion and gridding reconstruction to map all sensors to a virtual main camera, e.g., located at one main sensor like an RGB sensor, may be applied. If a depth image sensor is involved, a signal-conserving depth reconstruction algorithm can be applied, which may preferably give priority to proximal voxels in case of parallactic ambiguity in the short-axis dimension. Alternatively, reconstructing ambiguous voxels into a proximal and a, albeit sparsely filled, distal signal channel can be performed. Filling shadows and regions in left-right axis direction with ambiguous data with appropriate data using min-/max-pooling can be applied, or alternatively AI shadow filling can be used, which may be preferred over standard methods like signal nulling, or interpolation. A dynamic temporal alignment algorithm selecting the optimal data from the individual channels depending on the intended purpose and observed activity may be used. Alternatively, some sensors in the sensor arrangement may provide synchronization interfaces such as external trigger I/O for static temporal alignment. Figs. 3C and 3D show a proximal reconstructed shell giving proximal signals priority in ambiguous parallax situation occurring only along patient left-right axis. This ensures dense depth information also in the left-right direction and avoids artificial depth values. Fig. 3B show the result of this stable reconstruction of thin objects in the order of the sensor resolution of images acquired a sensor arrangement according to the invention and reconstructed via an appropriate algorithm. Thus, with a corresponding medical imaging or treatment system that utilizes a computed patient, exam and setup parameters for the examination or treatment, clinical workflow may be improved.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: sensor arrangement
- 110: plurality of sensors
- 120: first sensor
- 121: field of view of the first sensor
- 122: first axis
- 123: second axis
- 124: central axis
- 200: medical imaging or treatment system
- 210: patient bed
- 220: patient

## Claims

1. A sensor arrangement (100) for patient setup guidance to be embedded in a medical imaging or treatment system (200),
the sensor arrangement (100) comprising a plurality of sensors (110),
wherein at least a first sensor (120) of the plurality of sensors (110) comprises a field of view (121) of the first sensor (120) having an aspect ratio unequal to 1, thus comprising a first axis (122) of the field of view (121) of the first sensor (120) and a second axis (123) of the field of view (121) of the first sensor (120), wherein the field of view (121) of the first sensor (120) is larger in the direction of the first axis (122) than in the direction of the second axis (123), and
wherein the plurality of sensors (110) are arranged along a line parallel to the direction of the second axis (123) of the field of view (121) of the first sensor (120).

2. The sensor arrangement (100) according to claim 1, wherein the direction of the first axis (122) of the field of view of the first sensor (120) is perpendicular to the direction of the second axis (123) of the field of view of the first sensor (120).

3. The sensor arrangement (100) according to any one of the preceding claims, wherein a direction of a central axis (124) of the field of view (121) of the first sensor (120) is oriented essentially perpendicular to the first axis (122) and to the second axis (123).

4. The sensor arrangement (100) according to claim 3, wherein the central axis (124) corresponds to an imaging direction of the first sensor (120).

5. The sensor arrangement according (100) to any one of the preceding claims, wherein each sensor of the plurality of sensors (110) comprises a field of view having an aspect ratio unequal to 1, thus comprising a first axis of the field of view and a second axis of the field of view, wherein the field of view is larger in the direction of the first axis than in the direction of the second axis, and wherein each sensor of the plurality of sensors (110) is arranged along the line parallel to the direction of the second axis (123) of the field of view (121) of the first sensor (120) such that the first axis of the field of view of the respective sensor is parallel to the direction of the first axis (122) of the field of view (121) of the first sensor (120).

6. The sensor arrangement (100) according to any one of the preceding claims, wherein each one of the plurality of sensors (110) is selected from the group consisting of an optical camera, an RGB camera, a depth sensing device, a time-of-flight sensor, a radar sensor, a lidar sensor, a thermal sensor, or an infrared camera.

7. The sensor arrangement (100) according to claim 6, wherein each sensor of the plurality of sensors (110) is of a different type than any other sensor of the plurality of sensors (110).

8. The sensor arrangement (100) according to any one of the preceding claims, wherein the first sensor (120) is an RGB sensor.

9. The sensor arrangement (100) according to any one of the preceding claims, wherein the plurality of sensors (110) are arranged along the line parallel to the direction of the second axis (123) of the field of view (121) of the first sensor (120) such that a distance between each pair of adjacent sensors of the plurality of sensors (110) is minimized.

10. The sensor arrangement (100) according to claim 9, wherein the distance between each pair of adjacent sensors of the plurality of sensors (110) is smaller than 50 cm, preferably smaller than 30 cm, even more preferably smaller than 10 cm.

11. The sensor arrangement (100) according to any one of the preceding claims, wherein the aspect ratio of the field of view (121) of the first sensor (120) is larger than 1.2:1, preferably larger than 1.5:1; even more preferably larger than 2:1.

12. The sensor arrangement (100) according to any one of the preceding claims, further comprising a control unit configured for receiving data from each of the plurality of sensors (110) and mapping the received data to a virtual camera position, thereby providing a combined image.

13. A medical imaging or treatment system (200), comprising
a patient bed (210) configured for receiving a patient (220) for imaging or treatment; and
a sensor arrangement (100) according to any one of claims 1 to 12;
wherein the sensor arrangement (100) is positioned above the patient bed (210) and configured for imaging the patient (220); and
wherein the first axis (122) of the field of view (121) of the first sensor (120) is aligned parallel to a head-foot direction of the patient bed (210) and the second axis (123) of the field of view (121) of the first sensor (120) is aligned perpendicular to the head-foot direction of the patient bed (210) .

14. The medical imaging or treatment system (200) according to claim 13, wherein the sensor arrangement (100) is frontally facing the patient bed (210) and is configured for imaging the patient (220) with each sensor of the plurality of sensors (110).

15. The medical imaging or treatment system (200) according to any one of claims 13 or 14, wherein a field of view of each sensor of the plurality of sensors (110) encompasses the patient bed (210).
